# EUROPÄISCHE PATENTSCHRIFT

(11) **EP 0 809 700 B1**
(45) Veröffentlichungstag und Bekanntmachung des Hinweises auf die Patenterteilung: **10.05.2006**
(21) Anmeldenummer: 95934663.6
(22) Anmeldetag: 09.10.1995
(51) Int. Cl.: C12N 15/37, C12N 15/62, C07K 14/025, A61K 39/12

(54) **PAPILLOMAVIRUSÄHNLICHE PARTIKEL, FUSIONSPROTEINE SOWIE VERFAHREN ZU DEREN HERSTELLUNG**
PAPILLOMA VIRUS-LIKE PARTICLES, FUSION PROTEINS AND PROCESS FOR PRODUCING THE SAME
PARTICULES SEMBLABLES AU VIRUS DU PAPILLOME, PROTEINES DE FUSION ET LEUR PROCEDE DE PRODUCTION

(30) Priorität: 07.10.1994 DE 4435907; 21.07.1995 DE 19526752
(43) Veröffentlichungstag der Anmeldung: 03.12.1997
(73) Patentinhaber: LOYOLA UNIVERSITY OF CHICAGO, Chicago, IL 60611 (US)
(72) Erfinder: GISSMANN, Lutz, Willowbrook, IL 60514 (US); ZHOU, Jian, Willowbrook, IL 60514 (US); MÜLLER, Martin, Chicago, IL 60622 (US); PAINSTIL, Jeanette, Westchester, IL 60154 (US)
(74) Vertreter: UEXKÜLL & STOLBERG
(86) Internationale Anmeldenummer: PCT/EP1995/003974
(87) Internationale Veröffentlichungsnummer: WO 1996/011272

(56) Entgegenhaltungen:
- EP-A- 0 565 794
- WO-A-93/00436
- WO-A-93/02184
- WO-A-94/20137
- VIROLOGY, Bd. 194, 1993, Seiten 210-218, XP002001752 ZHOU J. ET AL.: "Glycosylation of human papillomavirus type 16 L1 protein"
- VIROLOGY, Bd. 170, 1989, Seiten 321-325, XP002001755 NASSERI M. ET AL.: "Genetic analysis of crpv pathogenesis : the open reading frame is dispensable for cellular transformation but is required for Pailloma formation"
- VIROLOGY, Bd. 178, 1990, Seiten 238-246, XP002001753 CRUM C.P. ET AL.: "Coexpression of the human papillomavirus type 16 e4 and l1 open reading frames in early cervical neoplasia"
- VIROLOGY, Bd. 185, 1991, Seiten 625-632, XP002001754 ZHOU J. ET AL.: "Identification of the nuclear localization signal of human papillomavirus type 16 l1 protein"

## Beschreibung

Die Erfindung betrifft rekombinant hergestellte papillomavirusähnliche Partikel, Proteine, Fusionsproteine sowie Verfahren zur Bildung und Reinigung dieser Partikel, Proteine und Fusionsproteine.

Infektionen mit bestimmten ("high-risk") Typen von genitalen Papillomaviren des Menschen (HPV), z.B. HPV 16, 18 oder 45 gelten als hauptsächlicher Risikofaktor für die Entstehung von bösartigen Tumoren des Anogenitaltrakts, von denen Gebärmutterhalskrebs (Cervixcarcinom) mit Abstand am häufigsten ist. Nach einer Schätzung der WHO treten jährlich weltweit etwa eine halbe Million neuer Fälle dieser Erkrankung auf. Aufgrund dieser Häufung ist der Zusammenhang zwischen HPV-Infektion und Cervixcarcinom am besten untersucht:
a) Vorläuferläsionen vom Cervixcarcinom (cervikale intraepitheliale Neoplasie: CIN) werden durch Papillomavirus-Infektion verursacht.
b) Die Genome bestimmter HPV-Typen (z.B. 16,18,33,35,45) werden in mehr als 95 % der Tumorbiopsien sowie in davon abgeleiteten Zellinien nachgewiesen. Abhängig vom geographischen Ursprung der Tumore enthalten 50 - 70 % davon HPV 16.
c) In allen daraufhin untersuchten Fällen werden die offenen Leseraster E6 und E7 transkribiert (Wettstein et al., in Pfister H. (ed): Papillomaviruses and human cancer, S. 155 bis 179, Boca Raton, 1990).
d) Die Proteine E6 und E7 sind in allen Cervixcarcinom-Zellinien sowie in *in vitro* transformierten menschlichen Keratinozyten nachweisbar und die Mehrzahl der Cervixcarcinom-Patientinnen haben E6- bzw. E7-spezifische Antikörper.
e) Die konstitutive Expression der E6/E7-Proteine ist notwendig zur Aufrechterhaltung des transformierten Zustandes HPV-positiver Tumore.
f) Die E6- und E7-Gene von HPV 16 und HPV 18 sind biologisch in folgenden experimentellen Systemen aktiv:
   - Induktion von zellulärer DNA Synthese in menschlichen Zellen;
   - Transformation von menschlichen Keratinozyten und anderen Zellen in Kultur;
   - Tumorbildung in transgenen Mäusen.

Andere HPV-Typen (in erster Linie HPV 6 und 11) verursachen gutartige Genitalwarzen (condylomata acuminata) und sind nur extrem selten mit bösartigen Tumoren assoziiert ("low-risk" Typen).

Genitale Papillomaviren des Menschen werden in der Regel durch Geschlechtsverkehr übertragen und führen in den meisten Fällen zu einer persistierenden Infektion in der Anogenital-Schleimhaut. Daraus wurde geschlossen, daß Primärinfektionen nur eine ungenügende Immunantwort induzieren oder daß das Virus Möglichkeiten entwickelt hat, in den infizierten Zellen der Immunüberwachung zu entkommen. Auf der anderen Seite gibt es gute Hinweise darauf, daß das Immunsystem bei der Primärmanifestation bzw. bei der malignen Progression von Papillomavirus-Infektionen beteiligt ist (zur Übersicht siehe Altmann et al. (1994) in Minson A., Neil J., McCrae M. (eds): Viruses and Cancer, Cambridge University Press, S. 71 bis 80).
a) Bei animalen Papillomaviren (Kaninchen-Papillomavirus und Rinder-Papillomavirus) läßt sich die klinische Manifestation von Primärinfektionen durch Vakzinierung mit Virus-Strukturproteinen oder mit Warzenextrakten ("autologe Vakzine") verhindern.
b) Nager werden durch Impfung mit HPV 16 E6- oder E7-positiven Vaccinia-Rekombinanten bzw. durch synthetische Peptide vor der Tumorbildung nach Inokulation HPV 16 transformierter autologer Zellen geschützt.
c) Regression von Warzen ist oftmals systemisch und läßt sich bei animalen Papillomaviren durch Transfer von Lymphozyten von "Regressor"-Tieren induzieren.
d) Die Häufigkeit von Genitalwarzen, CIN und Anogenitalkrebs ist bei immunsupprimierten Patienten (z.B. Nierentransplantierten oder HIV-Infizierten) erhöht.

Daraus wurde geschlossen, daß Papillomavirus-spezifische Impfungen mit dem Ziel der Verhinderung der Primärinfektion und der Entstehung von Genitalkrebs möglich sein sollten.

### 1. Geeignet ist die Verhinderung von HPV-Infektionen durch Impfung mit den Papillomavirus-Strukturproteinen L1 und L2 (prophylaktische Impfung).

Da sich Papillomaviren nicht in Zellkultur oder anderen experimentellen Systemen zu ausreichenden Titern vermehren lassen, können die viralen Proteine nur mit Hilfe rekombinanter Vektoren hergestellt werden. Kürzlich wurden virusähnliche Partikel (VLP), die nach Expression der viralen Strukturproteine L1 und L2 (bzw. L1 allein) in rekombinanten Vakzinia oder Baculovirus entstehen, beschrieben. Die Reinigung der VLP's ist sehr einfach mittels Zentrifugation in CsCl- oder Sucrosegradienten durchführbar.

WO 93/02184 beschreibt eine Methode, die "Papilloma virus like particles" (VLP's, Papillomavirus-ähnliche Partikel) zur Verfügung stellt, die für diagnostische Verwendungen oder als Vaccine gegen durch den Papillomavirus verursachte Infektionen genutzt werden. WO 94/20137 lehrt, dass nur L1-Protein zur Herstellung von VLP's benötigt wird. Dieses kann nach einer Mutation im 3' untranslatierten Bereich verstärkt exprimiert werden.

WO 94/00152 beschreibt ein rekombinant produziertes L1-Haupt-Capsid-Protein, welches die konformational neutralisierenden Epitope auf humanen und tierischen Papilloma-Virionen nachahmt (mimics). Diese rekombinanten Proteine sind als Vaccine, die gegen Papillomavirus-Infektionen schützen, einsetzbar. WO 93/00436 beschreibt ein Fusionsprotein, das L2-Protein oder Fragmente davon umfasst, zur Vaccinierung.

### 2. Behandlung von Cervixcarcinomen oder Vorläuferläsionen durch Immuntherapie mit Hilfe von frühen Papillomavirus-Proteinen (in erster Linie E6 bzw. E7), die in den persistent infizierten Zellen exprimiert werden (therapeutische Impfung).

Es wird angenommen, daß durch diese Impfung zytotoxische T-Zellen gegen persistent infizierte Genitalläsionen aktiviert werden. Zielpopulation sind Patienten mit HPVassoziierten prämalignen oder malignen Genitalläsionen.

Frühe HPV-Proteine werden durch Expression in E. coli oder eukaryontischen Vektoren (z.B. Baculovirus oder Hefe) hergestellt. Die Reinigung wird jedoch durch die geringe Löslichkeit erschwert und bedarf in der Regel einer Kombination von Ionenaustausch-Chromatographie, Gelfiltration und Affinitätschromatographie.

In der PCT-Anmeldung WO 93/20844 wird dargelegt, daß das E7-Protein des Papillomavirus aus HPV oder BPV therapeutisch effektiv in der Regression (jedoch nicht in der Prävention) von Papillomavirus-Tumoren in Säugern ist. Weiterhin werden auch bevorzugte antigene Protein-Fragment-Sequenzen beschrieben.

Bisher wurden jedoch keine VPL's beschrieben, die sich sowohl für die prophylaktische als auch die therapeutische Impfung eignen. Nachteilig ist bei den zuletzt genannten Verfahren, daß z.B. frühe HPV-Proteine aufgrund ihrer geringen Löslichkeit nur erschwert gereinigt werden können.

Besonders wünschenswert, insbesondere im Hinblick auf einen Impfstoff für die prophylaktische und therapeutische Impfung wäre eine hohe Partikelproduktion.

Nachteilig war an dem bisher beschriebenen Verfahren, daß die Herstellung von VLPs nach Expression von L1 in E. coli nicht möglich war.

Die Aufgabe der vorliegenden Erfindung ist es daher, rekombinant hergestellte Proteine sowie VLP's zur Verfügung zu stellen, die sich als Impfstoff zur prophylaktischen und therapeutischen Impfung eignen, sowie Verfahren zur Herstellung dieser Proteine und VLP's. Ebenso soll eine einfache Reinigung der erhaltenen rekombinanten Proteine ermöglicht sein. Auch sollte eine Herstellung von VLPs nach Expression von L1 in E. coli ermöglicht sein.

Die vorliegende Erfindung löst diese Aufgabe gemäß den vorliegenden unabhängigen Anprüchen. Weitere bevorzugte Ausgestaltungen, Aspekte und Details der Erfindung sind in den abhängigen Patentansprüchen der Beschreibung sowie den bevorzugten Ausführungsformen dargelegt.

Gemäß der vorliegenden Erfindung werden VLP's hergestellt, die aus Fusionsproteinen von späten und frühen HPV-Proteinen (oder Fragmenten davon) ("HVLP") bestehen und für prophylaktische bzw. therapeutische Impfung einsetzbar sind. Ein solcher Impfstoff besitzt gegenüber konventionellen Präparaten die im folgenden beschriebenen Vorteile:
a) Im Falle von prophylaktischer Impfung verhindern HVLP's durch Induktion L1/L2-spezifischer Antikörper nicht nur den Eintritt des Virus in die Zelle, sondern eliminieren bereits infizierte Zellen (durch Induktion von zytotoxischen T-Zellen), falls schon früher eine Infektion stattgefunden hat oder die humorale Immunantwort nicht ausreichend war.
b) Im Falle von therapeutischer Impfung eliminieren HVLP's persistent infizierte Zellen (z.B. bei Patienten mit CIN oder Cervixcarcinom), und verhindern vor allem bei Patientinnen mit CIN-Läsionen eine Reinfektion.
c) Die Reinigung der HVLP's ist ähnlich einfach wie die der VLP's ohne frühe HPV-Proteine.

Gemäß der vorliegenden Erfindung können VLP's des Bovinen papillomavirus (BPV) Typ 1 und der humanen Papillomaviren 11 und 16 nach Expression von L1 plus L2 bzw. von L1 allein in Vaccinia oder Baculovirus hergestellt werden. Experimente zeigen, daß Teile des L1-Proteins deletiert werden können (Aminosäuresequenz 311-351, 331-371, 391-431 von BPV 1; 306-315 von HPV 16), ohne daß die Fähigkeit zur Bildung von VLP's verloren geht. Solche Bereiche existieren in den L1-Proteinen aller Papillomaviren, so daß der deletierte Bereich von L1 durch andere Proteine (von Papillomaviren oder von anderem Ursprung) ersetzt werden kann und daß so virusähnliche Hybridpartikel hergestellt werden können.

Fusionsproteine, bestehend aus deletiertem L1-Protein von verschiedenen HPV-Typen (in erster Linie HPV 6, 11, 16, 18, 33, 35, 45) und den entsprechenden frühen Proteinen E1, E2, E4, E5, E6, E7 (oder Teilen davon) werden durch Expression in Vaccinia-Rekombinanten hergestellt, die in sehr kurzer Zeit konstruiert werden können. Die Bildung von VLP's, bestehend aus einem L1-Fusionsprotein wird durch Elektronenmikroskopie überprüft und das Vorhandensein des frühen HPV-Proteins durch Western Blot Analyse mit Hilfe spezifischer Antiseren getestet. Für die Produktion von HPLV's im großen Maßstab wird die Expression der Proteine in viralen oder eukaryotischen Systemen, bevorzugt in Baculovirus oder in Hefe, durchgeführt.

Entsprechende Experimente zur Herstellung von Fusionsproteinen können mit Proteinen anderen Ursprungs durchgeführt werden.

Wesentlich für die vorliegende Erfindung sind rekombinant hergestellte, virusähnliche Partikel (virus like particles, VLP's), die nach Expression der viralen Strukturproteine L1 und/oder L2 entstehen, wobei Abschnitte des L1-Proteins deletiert sind, ohne daß die Fähigkeit zur Bildung von VLP's verlorengeht.

Gemäß der vorliegenden Erfindung handelt es sich bei dem deletierten Bereich im L1-Protein des Bovinen Papillomavirus Typ 1 bevorzugt um die Aminosäuresequenzen 311-351, 331-371, 391-431. Bei L1-Proteinen des humanen Papillomavirus 16 handelt es sich vorteilhafterweise um die Aminosäuresequenz 306-315.

In einer bevorzugten Ausgestaltung der vorliegenden Erfindung wird der deletierte Bereich von L1- Proteinen durch andere Proteine oder Proteinfragmente ersetzt, wobei Fusionsproteine erhalten werden. Der Anteil an L1-Protein beträgt vorteilhafterweise ca. 50 bis 99 %, bevorzugt ca. 60 bis 90 %, besonders bevorzugt ca. 80 %.

Gemäß der vorliegenden Erfindung sollen jedoch, wenn auch im weiteren nicht explizit erwähnt, auch mehr als ein Bereich, des L1- Proteins deletiert und bevorzugt durch andere Proteine oder Proteinfragmente ersetzt werden.

Besonders bevorzugt wird der deletierte Bereich im L1-Protein durch andere Proteine von Papillomaviren und/oder Proteine anderen Ursprungs ersetzt, wodurch virusähnliche Hybridpartikel (HVLP's) herstellbar sind.

Es hat sich als besonders vorteilhaft gemäß der vorliegenden Erfindung erwiesen, daß die Bildung der VLP's aus einem L1-Fusionsprotein erfolgt.

Die Fusionsproteine, insbesondere zur Bildung von virusählichen Hybridpartikeln gemäß einer weiteren Ausgestaltung der vorliegenden Erfindung, bestehen vorteilhafterweise aus einem deletierten L1-Protein von verschiedenen HPV-Typen (human papilloma virus), besonders bevorzugt HPV 6, 11, 16, 18, 33, 35 und 45, und anderen Proteinen oder Proteinfragmenten. Bevorzugt handelt es sich bei diesen anderen Proteinen oder Proteinfragmenten um entsprechende frühe Proteine oder Fragmente davon, wie z.B. die frühen Proteine E1, E2, E4, E5, E6 und/oder E7.

Gemäß dem erfindungsgemäßen Verfahren wird die Expression der Fusionsproteine und Proteine in viralen oder eukaryotischen Vektoren, ganz besonders bevorzugt in Baculoviren oder in Hefen, durchgeführt.

Gemäß einer weiteren Ausgestaltung des erfindungsgemäßen Verfahrens werden die Fusionsproteine durch Expression in Vaccinia-Rekombinanten hergestellt.

Die Anwendung der Fusionsproteine oder der virusähnlichen Hybridpartikel zur Herstellung eines prophylaktischen und therapeutischen Impfstoffes erfolgt gemäß der vorliegenden Erfindung bevorzugt nach Zugabe weiterer Komponenten.

Bislang wurde zur Herstellung von VLPs, wie z.B. von VLPs aus HPV 16, das L1 offene Leseraster (ORF) mit Hilfe eukaryontischer Vektoren, wie z.B. Bakulovirus, exprimiert. Die Bildung der VLPs (Assembly) erfolgt im Zellkern der infizierten Zellen.

Wesentlich für die vorliegende Erfindung sind deshalb insbesondere rekombinant hergestellte, papillomavirusähnliche Partikel, die nach Expression der viralen Strukturproteine L1 und/oder L2 entstehen, in denen ein oder mehrere Abschnitte des L1- Proteins deletiert sind, wobei die Fähigkeit zur Bildung von virusähnlichen Partikeln im Vergleich zur nativen Bildung und/oder *in vitro* Produktion erhöht ist.

Gemäß der vorliegenden Erfindung handelt es sich bei mindestens einem der deletierten Bereiche im L1-Protein eines Papillomavirus um eine Deletion, vorteilhafterweise in der C-terminalen Aminosäuresequenz, bevorzugt in einer Länge von ungefähr 1 bis 34 Aminosäuren (AS), bevorzugt von 1 bis 26 Aminosäuren (AS), insbesondere von 26 AS.

Vorteilhafterweise wird nach Einfügen der C-terminalen Deletion in das L1- Protein die Produktion von VLPs um ein Vielfaches erhöht, bevorzugt um das mindestens 10-fache, und insbesondere um das ungefähr 10- bis 100-fache.

In einer bevorzugten Ausgestaltung der vorliegenden Erfindung handelt es sich bei den deletierten Bereichen im L1-Protein, insbesondere des Bovinen Papillomavirus, um 26 C-terminale Aminosäuren. Besonders bevorzugt ist die 26 AS große, C-terminale Deletion (Gly-Ala-Gly-Cys-Ser-Thr-Val-Arg-Lys-Arg-Arg-Ile-Ser-Gln-Lys-Thr-Ser-Ser-Lys-Pro-Ala-Lys-Lys-Lys-Lys-Lys) entsprechend der Nucleotid-Position 7016 bis 7093 GGGGCAGGAT GTTCAACTGT GAGAAAACGA AGAATTAGCC AAAAAACTTC CAGTAAGCCT GCAAAAAAAA AAAAAAAA in das L1 ORF des Bovinen Papillomavirus Typ 1 (BPV 1) eingefügt. Vorteilhafterweise ist nach Einfügen der C-terminalen Deletion in das L1-Protein die Produktion von VLP's um das mindestens 10-fache gesteigert.

Gemäß einer weiteren Ausgestaltung der vorliegenden Erfindung handelt es sich bei der mindestens einen Deletion im L1-Protein um eine homologe Aminosäuresequenz des humanen Papillomavirus 15 oder anderer Papillomaviren.

Gemäß einer weiteren bevorzugten Ausgestaltung handelt es sich bei den deletierten Bereichen im L1-Protein um 34 C-terminale Aminosäuren des Humanen Papillomavirus Typ 16 (HPV 16), bevorzugt um die AS-Sequenz Ala-Gly-Leu-Lys-Ala-Lys-Pro-Lys-Phe-Thr-Leu-Gly-Lys-Arg-Lys-Ala-Thr-Pro-Thr-Thr-Ser-Ser-Thr-Ser-Thr-Thr-Ala-Lys-Arg-Lys-Lys-Arg-Lys-Leu) entsprechend der Nucleotid-Position 7052 bis 7153 GCAGGATTGA AGGCCAAACC AAAATTTACA TTAGGAAAAC GAAAAGCTAC ACCCACCACC TCATCTACCT CTACAACTGC TAAACGCAAA AAACGTAAGC TG, welche in das L1 ORF des HPV 16 eingefügt ist.

Besonders bevorzugt umfaßt die Deletion des L1-Proteins das nukleäre Lokalisationssignal (NLS). Die Partikelproduktion aus den L1-Proteinen oder den L1-Proteinen und L2-Proteinen erfolgt insbesondere im Zytoplasma. Bevorzugt werden die Partikel in den Überstand sezerniert, besonders bevorzugt ist eine Sekretion von ungefähr 5 bis 10 % der Partikel.

Die Expression von L1-Proteinen oder L1-Proteinen und L2-Proteinen in E. coli erfolgt gemäß einer weiteren bevorzugten Ausführungsform. Hierbei sind an der C-terminalen Deletion im L1-Protein insbesondere zusätzlich 6 Histidine eingefügt. Vorteilhafterweise erfolgt die Herstellung von VLP's nach Expression von L1-Proteinen oder L1- und L2-Proteinen in E. coli.

Gemäß der vorliegenden Erfindung handelt es sich bei den weiteren deletierten Bereichen im L1-Protein des Bovinen Papillomavirus Typ 1 bevorzugt um die Aminosäuresequenzen 311-351, 331-371, 391-431. Bei L1-Proteinen des humanen Papillomavirus 16 handelt es sich vorteilhafterweise um die Aminosäuresequenz 306-315.

In einer bevorzugten Ausgestaltung der vorliegenden Erfindung wird der weitere deletierte Bereich von L1-Proteinen durch andere Proteine oder Proteinfragmente ersetzt, wobei Proteine erhalten werden, die im weiteren als Fusionsproteine bezeichnet werden. Der Anteil an L1-Protein beträgt vorteilhafterweise ca. 50 bis 99 %, bevorzugt ca. 60 bis 90 %, besonders bevorzugt ca. 80 %.

Gemäß der vorliegenden Erfindung sollen jedoch, wenn auch im weiteren nicht explizit erwähnt, auch mehr als ein weiterer Bereich des L1-Proteins deletiert und bevorzugt durch andere Proteine oder Proteinfragmente ersetzt werden.

Besonders bevorzugt wird der deletierte Bereich von L1-Protein durch andere Proteine von Papillomaviren und/oder Proteine anderen Ursprungs ersetzt, wodurch virusähnliche Hybridpartikel (HVLP's) herstellbar sind.

Es hat sich als besonders vorteilhaft gemäß der vorliegenden Erfindung erwiesen, daß die Bildung der V-LP's aus einem L1-Protein, einem L1-Fusionsprotein, einem L1-Protein und L2-Protein, einem L1-Fusionsprotein und L2-Protein, einem L1-Protein und einem L2-Fusionsprotein oder einem L1-Fusionsprotein und einem L2-Fusionsprotein erfolgt, wobei ein sehr mehrere Abschnitte des L1-Proteins deletiert sind. Gemäß einer weiteren Ausgestaltung der vorliegenden Erfindung handelt es sich bei mindestens einem der deletieren Bereiche im L1- Protein eines Papillomavirus um N-terminale Aminosäuresequenzen.

Gemäß der vorliegenden Erfindung handelt es sich bei einer weiteren Ausführungsform bei mindestens einem der deletierten Bereiche im L1-Protein Protein eines Papillomavirus um Aminosäuresequenzen im mittleren Bereich des Proteins.

Wesentlich für die Erfindung sind ebenfalls Proteine, insbesondere zur Bildung von papillomavirusähnlichen Hybridpartikeln, wobei ein oder mehrere Abschnitte des L1-Proteins deletiert sind. Insbesondere handelt es sich bei mindestens einem der deletierten Bereiche im L1-Protein um eine Deletion einer C-terminalen Aminosäuresequenz.

Die Fusionsproteine, insbesondere zur Bildung von papillomavirusähnlichen Hybridpartikeln gemäß einer weiteren Ausgestaltung der vorliegenden Erfindung, bestehen vorteilhafterweise aus einem deletierten L1-Protein von verschiedenen Papillomaviren, besonders bevorzugt HPV 6, 11, 16, 18, 31, 33, 35 und 45, und anderen Proteinen oder Proteinfragmenten von Papillomaviren oder von anderer Herkunft. Bevorzugt handelt es sich bei diesen anderen Proteinen oder Proteinfragmenten um entsprechende frühe Papillomavirus-Proteine oder Fragmente davon, wie z.B. die frühen Proteine E1, E2, E4, E5, E6 und/oder E7.

Gemäß dem erfindungsgemäßen Verfahren wird zur Expression der Proteine und/oder Fusionsproteine und Produktion von papillomavirusähnlichen Partikeln in viralen, eukaryotischen oder prokaryotischen Vektoren, ganz besonders bevorzugt in Vaccinia-Rekombinanten, in Baculoviren, in Hefen oder in Bakterien, insbesondere in E. coli, durchgeführt.

Bevorzugt erfolgt die Partikelproduktion im Zytoplasma. Die Partikel werden besonders bevorzugt in den Überstand sezerniert, ganz besonders bevorzugt werden ungefähr 5 bis 10 % der Partikel in den Überstand sezerniert.

Insbesondere wird gemäß der vorliegenden Erfindung durch Einfügen einer 26 Aminosäure (AS) großen, C-terminalen Deletion in der Nucleotidposition 7016-7093 in das L1 ORF des bovinen Papillomavirus Typ 1 (BPV 1) die Produktion von VLPs um das mehr als 10-fache gesteigert. So läßt sich bei gleicher Menge an L1-Protein, wie z.B. in einem Western Blot demonstrierbar, eine Steigerung der Partikelzahl im Elektronenmikroskop zeigen. Da die Deletion bevorzugt das nukleäre Lokalisationssignal (NLS) umfaßt, erfolgt die Partikelproduktion im Zytoplasma, ein erheblicher Teil der Partikel wird in den Überstand sezerniert. Dies ist besonders vorteilhaft, da hierdurch die Reinigung wesentlich erleichtert wird.

Proteine, bevorzugt mit genannter Deletion mit zusätzlichen 6 Histidinen (His-L1-Proteine) werden gemäß der vorliegenden Erfindung in E. coli exprimiert. Die Proteine, insbesondere His-L1-Proteine, werden bevorzugt über Ni-Affinitätschromatograpie gereinigt, wobei die Proteine entsprechend einer vorteilhaften Ausführungs form zu diesem Zeitpunkt in Denaturierungspuffer, beispielsweise 6 M Guanidinhydrochlorid, vorliegen. Die Renaturierung erfolgt beispielsweise in 150 mM NaCl, 1 mM CaCl₂, 0,01 % Triton-X 100, 10 mM Hepes (N-2-hydroxyethylpiperazine-N'-2-ethansulfonsäure), pH 7,4.

Die Produktion (Assembly) der VLP erfolgt gemäß einer bevorzugten Ausgestaltung der vorliegenden Erfindung nach Dialyse der Proteine, vorteilhafterweise nach Dialyse gegen 150 mM NaCl, 25 mM Ca²⁺, 10 % DMSO (Dimethylsulfoxid), 0,1 % Triton-X 100, 10 mM Tris [tris-(hydroxymethyl)aminomethan] Essigsäure bei einem pH-Wert von 5,0.

Die Deletion von Sequenzen im L1-Protein von sämtlichen Papillomaviren, die das vorzeitige Assembly der VLPs verhindern, führt zu einer höheren Ausbeute bei der VLP Produktion.

Sofern in diesen Fällen das L1 NLS betroffen ist, erfolgt die Assembly im Zytoplasma. Somit ist die Reinigung des VLPs erfindungsgemäß aus dem Zytoplasma, statt wie bisher aus dem Zellkern, möglich. Erfindungsgemäß sind auch kürzere Deletionen möglich. Gemäß der vorliegenden Erfindung werden Deletionen bis zu einer Aminosäure und/oder Substitutionen von bis zu einer Aminosäure durchgeführt. Hierbei erweist es sich als vorteilhaft, daß bei kurzen Deletionen bzw. bei Substitutionen von bis zu einer bzw. nur weniger Aminosäuren die antigenen Eigenschaften der Proteine und der sich daraus gebildeten VLPs so wenig wie möglich gegenüber der nativen antigenen Eigenschaft der Proteine bzw. VLPs verändert sind.

Die Einführung einer wie vorgehend ausgeführten C-terminalen Deletion bzw. Substitution in L1-Fusionsproteine, führt auch zu einer Steigerung der Produktion von Hybrid VLPs. Hierbei sollen auch die VLPs eingeschlossen sein, die nur L1-Fusionsproteine enthalten, sowie Hybrid VLPs, die ein L1- Fusionsprotein und ein L2- bzw. L1-Protein enthalten.

Hierzu werden VLP's hergestellt, die aus Fusionsproteinen von späten und frühen HPV-Proteinen (oder Fragmenten davon) ("HVLP") bestehen und für prophylaktische bzw. therapeutische Impfung einsetzbar sind. Ein solcher Impfstoff besitzt gegenüber konventionellen Präparaten die im folgenden beschriebenen Vorteile:
a) Im Falle von prophylaktischer Impfung verhindern HVLP's durch Induktion L1/L2-spezifischer Antikörper nicht nur den Eintritt des Virus in die Zelle, sondern eliminieren bereits infizierte Zellen (durch Induktion von zytotoxischen T-Zellen), falls schon früher eine Infektion stattgefunden hat oder die humorale Immunantwort nicht ausreichend war.
b) Im Falle von therapeutischer Impfung eliminieren HVLP's persistent infizierte-Zellen ( z.B. bei Patienten mit CIN oder Cervixcarcinom), und verhindern vor allem bei Patientinnen mit CIN-Läsionen eine Reinfektion.
c) Die Reinigung der HVLP's ist ähnlich einfach wie die der VLP's ohne frühe HPV-Proteine.

VLP's des Bovinen Papillomavirus (BPV) Typ 1 und der humanen Papillomaviren 11 und 16 können nach Expression von L1 plus L2 bzw. von L1 allein in Vaccinia oder Baculovirus hergestellt werden. Experimente zeigen, daß Teile des L1-Proteins deletiert werden können (Aminosäuresequenz 311-351, 331-371, 391-431 von BPV 1; 306-315 von HPV 16), ohne daß die Fähigkeit zur Bildung von VLP's verloren geht. Solche Bereiche existieren in den L1-Proteinen aller Papillomaviren, so daß der deletierte Bereich von L1 durch andere Proteine (von Papillomaviren oder von anderem Ursprung) ersetzt werden kann und daß so virusähnliche Hybridpartikel hergestellt werden können.

Fusionsproteine bestehend aus deletiertem L1- Protein von verschiedenen HPV-Typen (in erster Linie HPV 6, 11, 16, 18, 33, 35, 45) und den entsprechenden frühen Proteinen E1, E2, E4, E5, E6, E7 (oder Teilen davon) werden durch Expression in Vaccinia-Rekombinanten hergestellt, die in sehr kurzer Zeit konstruiert werden können. Die Bildung von VLPs, bestehend aus einem L1-Fusionsprotein wird durch Elektronenmikroskopie überprüft und das Vorhandensein des frühen HPV-Proteins durch Western Blot Analyse mit Hilfe spezifischer Antiseren getestet. Für die Produktion von HPLV's im großen Maßstab wird die Expression der Proteine in viralen eukaryotischen oder prokaryotischen Systemen, bevorzugt in Baculovirus, in Hefe, oder in E. coli durchgeführt.

Die Anwendung der Fusionsproteine oder der virusähnlichen Hybridpartikel zur Herstellung eines prophylaktischen und therapeutischen Impfstoffs erfolgt gemäß der vorliegenden Erfindung bevorzugt nach Zugabe weiterer Komponenten.

Entsprechende Experimente zur Herstellung von Fusionsproteinen können mit Proteinen anderen Ursprungs durchgeführt werden.

## Patentansprüche

1. Rekombinant hergestellte papillomavirusähnliche Partikel, die nach Expression der viralen Strukturproteine L1 oder L1 und L2 entstehen, **dadurch gekennzeichnet, dass** ein oder mehrere Abschnitte des L1- -Proteins deletiert sind, wobei die Fähigkeit zur Bildung von virusähnlichen Partikeln bestehen bleibt.

2. Virusähnliche Partikel nach Anspruch 1, **dadurch gekennzeichnet, dass** es sich bei den deletierten Bereichen im L1-Protein um die Aminosäuresequenzen 311-351, 331-371, 391-431 des Bovinen Papillomavirus Typ 1 handelt.

3. Virusähnliche Partikel nach Anspruch 1, **dadurch gekennzeichnet, dass** es sich bei den deletierten Bereichen im L1-Protein um die Aminosäuresequenz 306-315 des humanen Papilloma-virus 16 handelt.

4. Rekombinant hergestellte papillomavirusähnliche Partikel, die nach Expression der viralen Strukturproteine L1 oder L1 und L2 entstehen,
**dadurch gekennzeichnet, dass** ein oder mehrere Abschnitte des L1- Proteins deletiert sind, wobei die Fähigkeit zur Bildung von virusähnlichen Partikeln im Vergleich zur nativen Bildung und/oder in vitro Produktion erhöht ist.

5. Virusähnliche Partikel nach Anspruch 4, **dadurch gekennzeichnet, dass** es sich bei mindestens einem der deletierten Bereiche im L1- Protein eines Papillomavirus um C-terminale Aminosäuresequenzen handelt.

6. Virusähnliche Partikel nach Anspruch 5, **dadurch gekennzeichnet, dass** es sich bei dem mindestens einen deletierten Abschnitt im C-terminalen Bereich im L1- Protein um eine Aminosäuresequenz in einer Länge von ungefähr 1 bis 34 Aminosäuren, bevorzugt 1 bis 26 Aminosäuren, eines Papillomavirus handelt.

7. Virusähnliche Partikel nach einem der Ansprüche 4 bis 6, **dadurch gekennzeichnet, dass** nach Einfügen insbesondere der C-terminalen Deletion in das L1- Protein die Produktion von VLPs um ein Vielfaches, bevorzugt um das mindestens 10-fache, besonders bevorzugt um das ca. 10- bis 100-fache, gesteigert wird.

8. Virusähnliche Partikel nach einem der Ansprüche 4 bis 7, **dadurch gekennzeichnet, dass** es sich bei den deletierten Bereichen in L1- Protein um 26 C-terminale Aminosäuren des Bovinen Papillomavirus handelt.

9. Virusähnliche Partikel nach Anspruch 8, **dadurch gekennzeichnet, dass** die 26 AS große, C-terminale Deletion
(Gly-Ala-Gly-Cys-Ser-Thr-Val-Arg-Lys-Arg-Arg-Ile-Ser-Gln-Lys-Thr-Ser-Ser-Lys-Pro-Ala-Lys-Lys-Lys-Lys-Lys) entsprechend der Nucleotid-Position 7016 bis 7093 GGGGCAGGAT GTTCAACTGT GAGAAAACGA AGAATTAGCC AAAAAACTTC CAGTAAGCCT GCAAAAAAAA AAAAAAAA, in das L1 ORF des Bovinen Papillomavirus Typ 1 eingefügt ist.

10. Virusähnliche Partikel nach einem der Ansprüche 4 bis 7, **dadurch gekennzeichnet, dass** es sich bei den deletierten Bereichen im L1- Protein um 34 C-terminale Aminosäuren des humanen Papillomavirus Typ 16 (HPV 16) handelt.

11. Virusähnliche Partikel nach Anspruch 10, **dadurch** gekennzeichet, dass die 34 AS große, C-terminale Deletion
(Ala-Gly-Leu-Lys-Ala-Lys-Pro-Lys-Phe-Thr-Leu-Gly-Lys-Arg-Lys-Ala-Thr-Pro-Thr-Thr-Ser-Ser-Thr-Ser-Thr-Thr-Ala-Lys-Arg-Lys-Lys-Arg-Lys-Leu) entsprechend der Nucleotid-Position 7052 bis 7153 GCAGGATTGA AGGCCAAACC AAAATTTACA TTAGGAAAAC GAAAAGCTAC ACCCACCACC TCATCTACCT CTACAACTGC TAAACGCAAA AAACGTAAGC TG in das L1 ORF des Humanen Papillomavirus Typ 16 (HPV 16) eingefügt ist.

12. Virusähnliche Partikel nach einem der Ansprüche 5 bis 7, **dadurch** gekennzeichet, dass es sich bei der mindestens einen Deletion im C-terminalen Bereich im L1- Protein um eine homologe Aminosäuresequenz des humanen Papillomavirus 16 oder anderer Papillomaviren handelt.

13. Virusähnliche Partikel nach einem der Ansprüche 4 bis 12, **dadurch gekennzeichnet, dass** die Deletion des L1- Proteins das nukleäre Lokalisationssignal (NLS) umfasst.

14. Virusähnliche Partikel nach einem der Ansprüche 4 bis 13, **dadurch gekennzeichnet, dass** die Partikelproduktion aus den L1-Proteinen oder L1- und L2-Proteinen im Zytoplasma erfolgt.

15. Virusähnliche Partikel nach einem der Ansprüche 4 bis 14, **dadurch gekennzeichnet, dass**-Partikel in den Überstand sezerniert werden.

16. Virusähnliche Partikel nach Anspruch 15, **dadurch gekennzeichnet, dass** ca. 5 bis 10 % der Partikel in den Überstand sezerniert werden.

17. Virusähnliche Partikel nach einem der Ansprüche 4 bis 16, **dadurch gekennzeichnet, dass** die Expression von L1-Proteinen oder L1- und L2-Proteinen in E. coli erfolgt.

18. Virusähnliche Partikel nach Anspruch 17, **dadurch gekennzeichnet, dass** an der C-terminalen Deletion im L1-Protein 6 Histidine eingefügt wurden.

19. Virusähnliche Partikel nach einem der Ansprüche 17 bis 18, **dadurch gekennzeichnet, dass** die Herstellung von VLPs nach Expression von L1-Proteinen oder L1- und L2-Proteinen in E. coli erfolgt.

20. Virusähnliche Partikel nach einem der Ansprüche 4 bis 19, **dadurch gekennzeichnet, dass** es sich bei den weiteren deletierten Bereichen im L1-Protein um die Aminosäuresequenzen 311-351, 331-371, 391-431 des Bovinen Papillomavirus Typ 1 handelt.

21. virusähnliche Partikel nach Anspruch 20, **dadurch gekennzeichnet, dass** es sich bei den weiteren deletierten Bereichen im L1-Protein um die Aminosäuresequenz 306-als des humanen Papillomavirus 16 handelt.

22. Virusähnliche Partikel nach einem der Ansprüche 1 bis 21, **dadurch gekennzeichnet, dass** der deletierte Bereich von L1-Proteinen durch andere Proteine oder Proteinfragmente ersetzt ist, um ein Fusionsprotein zu erhalten.

23. Virusähnliche Partikel nach Anspruch 22, **dadurch gekennzeichnet, dass** der Anteil an L1- Protein ca. 50 - 99 %, bevorzugt ca. 60 bis 90 %, besonders bevorzugt ca. 80 % des Fusionsproteins beträgt.

24. Virusähnliche Partikel nach Anspruch 22 und 23, **dadurch gekennzeichnet, dass** der deletierte Bereich im L1- Protein durch andere Proteine von Papillomaviren und/oder Proteinen anderen Ursprungs ersetzt ist, wodurch virusähnliche Hybridpartikel herstellbar sind.

25. Virusähnliche Partikel nach einem der Ansprüche 1 bis 24, **dadurch gekennzeichnet, dass** die Bildung der virusähnlichen Partikel aus einem L1-Protein, einem L1-Fusionsprotein, einem L1-Protein und L2-Protein, einem L1-Fusionsprotein und L2-Protein, einem L1-Protein und einem L2-Fusionsprotein oder einem L1-Fusionsprotein und einem L2-Fusionsprotein erfolgt.

26. Virusähnliche Partikel nach einem der Ansprüche 4 bis 25, **dadurch gekennzeichnet, dass** es sich bei mindestens einem der deletierten Bereiche im L1- Protein eines Papillomavirus um N-terminale Aminosäuresequenzen handelt.

27. Virusähnliche Partikel nach einem der Ansprüche 4 bis 26, **dadurch gekennzeichnet, dass** es sich bei mindestens einem der deletierten Bereiche im L1- Protein eines Papillomavirus um Aminosäuresequenzen im mittleren Bereich des Proteins handelt.

28. Fusionsproteine, zur Bildung von papillomavirusähnlichen Hybridpartikeln nach einem der Ansprüche 22 bis 25, **dadurch gekennzeichnet, dass** die Fusionsproteine deletierte L1-Proteine von verschiedenen Papillomaviren und andere Proteine oder Fragmente von Papillomaviren oder anderer Herkunft enthalten.

29. Fusionsproteine, zur Bildung von papillomavirusähnlichen Hybridpartikeln nach einem der Ansprüche 22 bis 25, wobei die Fusionsproteine deletierte L1- Proteine von verschiedenen Papillomaviren und andere Proteine oder Fragmente von Papillomaviren oder anderer Herkunft enthalten, **dadurch gekennzeichnet, dass** die anderen Proteine oder Fragmente frühe Papillomavirus-Proteine oder Fragmente davon sind.

30. Fusionsprotein nach Anspruch 28 oder 29, **dadurch gekennzeichnet, dass** es sich um die frühen Proteine E1, E2, E4, E5, E6, E7 oder um Fragmente davon handelt.

31. Fusionsproteine nach einem der Ansprüche 28 bis 30, **dadurch gekennzeichnet, dass** es sich um deletierte L1-Proteine von verschiedenen HPV-Typen wie HPV 6, 11, 16, 18, 31, 33, 35, 45 handelt.

32. Verfahren zur Expression der Fusionsproteine gemäß einem der Ansprüche 28 bis 31, **dadurch gekennzeichnet, dass** die Expression der Proteine und Fusionsproteine in viralen oder in eukaryotischen Vektoren durchgeführt wird.

33. Verfahren zur Expression der Fusionsproteine und Produktion von papillomavirusähnlichen Partikeln gemäß einem der Ansprüche 4 bis 31, **dadurch gekennzeichnet, dass** die Expression der L1 und/oder L2-Proteine und Fusionsproteine in viralen, eurkaryotischen oder prokaryotischen Vektoren durchgeführt wird.

34. Verfahren nach einem der Ansprüche 32 oder 33, **dadurch gekennzeichnet, dass** die L1 und/oder L2-Proteine oder Fusionsproteine durch Expression in Vaccinia-Rekombinanten hergestellt werden.

35. Verfahren nach einem der Ansprüche 32 oder 33, **dadurch gekennzeichnet, dass** die Expression der L1 und/oder L2-Proteine und Fusionsproteine in Baculoviren oder in Hefen durchgeführt wird.

36. Verfahren nach Anspruch 33, **dadurch gekennzeichnet, dass** die Expression der L1 und/oder L2-Proteine und Fusionsproteine in E. coli durchgeführt wird.

37. Verfahren nach Anspruch 36, **dadurch gekennzeichnet, dass** die Reinigung, insbesondere der L1-Proteine, durch Ni-Affinitätschromatographie und Renaturierung, bevorzugt in 150 mM NaCl, 1 mM CaCl₂, 0,01 % Triton-X 100, 10 mM Hepes pH 7,4, erfolgt.

38. Verfahren nach Anspruch 37, **dadurch gekennzeichnet, dass** die Proteine zum Zeitpunkt der Reinigung in Denaturierungspuffer vorliegen.

39. Verfahren nach Anspruch 38, **dadurch gekennzeichnet, dass** es sich bei dem Denaturierungspuffer um 6 M Guanidinhydrochlorid handelt.

40. Verfahren nach einem der Ansprüche 33 bis 39, **dadurch gekennzeichnet**, das das Assembly der VLPs nach Dialyse erfolgt.

41. Verfahren nach Anspruch 40, **dadurch gekennzeichnet, dass** die Dialyse gegen 150 mM NaCl, 25 mM Ca²⁺, 10 DMSO, 0,1 % Triton-X 100 und 10 mM Tris Essigsäure bei pH = 5,0 erfolgt.

42. Verfahren nach einem der Ansprüche 33 bis 41, **dadurch gekennzeichnet, dass** die Partikelproduktion im Zytoplasma erfolgt.

43. Verfahren nach einem der Ansprüche 33 bis 42, **dadurch gekennzeichnet, dass** Partikel in den Überstand sezerniert werden.

44. Verfahren nach Anspruch 43, **dadurch gekennzeichnet, dass** ungefähr 5 bis 10 % der Partikel in den Überstand sezerniert werden.

45. Verwendung der Fusionsproteine gemäß einem der Ansprüche 28 bis 31 zur Herstellung eines Impfstoffs zur prophylaktischen und/oder therapeutischen Impfung, bevorzugt nach Zugabe weiterer Komponenten.

46. Verwendung der virusähnlichen Hybridpartikel gemäß einem der Ansprüche 1 bis 27 zur Herstellung eines Impfstoffs zur prophylaktischen und/oder therapeutischen Impfung, bevorzugt nach Zugabe weiterer Komponenten.

## Claims

1. Recombinant-produced papilloma virus-like particles that are formed after expression of the viral structure proteins L1 or L1 and L2, **characterised in that** one or more sections of the L1 protein are deleted, wherein the ability to form virus-like particles remains.

2. Virus-like particles according to claim 1,
**characterised in that** the deleted regions in the L1 protein concern the amino acid sequences 311-351, 331-371,391-431 of bovine papilloma virus type 1.

3. Virus-like particles according to claim 1, **characterised in that** the deleted regions in the L1 protein concern the amino acid sequence 306-315 of the human papilloma virus 16.

4. Recombinant-produced papilloma virus-like particles that are formed after expression of the viral structure proteins L1 or L1 and L2, **characterised in that** one or more sections of the L1 protein are deleted, wherein the ability to form virus-like particles is increased compared to the native formation and/or *in vitro* production.

5. Virus-like particles according to claim 4, **characterised in that** at least one of the deleted regions in the L1 protein of a papilloma virus concerns C-terminal amino acid sequences.

6. Virus-like particles according to claim 5, **characterised in that** the at least one deleted section in the C-terminal region in the L1 protein concerns an amino acid sequence having a length of about 1 to 34 amino acids, preferably 1 to 26 amino acids, of a papilloma virus.

7. Virus-like particles according to one of claims 4 to 6, **characterised in that** after insertion in particular of the C-terminal deletion into the L1 protein the production of virus-like particles is increased by a multiple, preferably by at least 10 times, particularly preferably by ca. 10 times to 100 times.

8. Virus-like particles according to one of claims 4 to 7, **characterised in that** the deleted regions in the L1 protein concern 26 C-terminal amino acids of bovine papilloma virus.

9. Virus-like particles according to claim 8, **characterised in that** the 26 amino acid long, C-terminal deletion (Gly-Ala-Gly-Cys-Ser-Thr-Val-Arg-Lys-Arg-Arg-Ile-Ser-Gln-Lys-Thr-Ser-Ser-Lys-Pro-Ala-Lys-Lys-Lys-Lys-Lys) corresponding to the nucleotide position 7016 to 7093 GGGGCAGGAT GTTCAACTGT GAGAAAACGA AGAATTAGCC AAAAAACTTC CAGTAAGCCT GCAAAAAAAA AAAAAAAA is introduced into the L1 ORF of bovine papilloma virus type 1.

10. Virus-like particles according to one of claims 4 to 7, **characterised in that** the deleted regions in the L1 protein concern 34 C-terminal amino acids of human papilloma virus type 16 (HPV 16).

11. Virus-like particles according to claim 10, **characterised in that** the 34 amino acid long, C-terminal deletion (Ala-Gly-Leu-Lys-Ala-Lys-Pro-Lys-Phe-Thr-Leu-Gly-Lys-Arg-Lys-Ala-Thr-Pro-Thr-Thr-Ser-Ser-Thr-Ser-Thr-Thr-Ala-Lys-Arg-Lys-Lys-Arg-Lys-Leu) corresponding to the nucleotide position 7052 to 7153 GCAGGATTGA AGGCCAAACC AAAATTTACA TTAGGAAAAC GAAAAGCTAC ACCCACCACC TCATCTACCT CTAGAACTGC TAAACGCAAA AAACGTAAGC TG is introduced into the L1 ORF of human papilloma virus type 16 (HPV 16).

12. Virus-like particles according to one of claims 5 to 7, **characterised in that** the at least one deletion in the C-terminal region in the L1 protein concerns an homologous amino acid sequence of human papilloma virus 16 or other papilloma viruses.

13. Virus-like particles according to one of claims 4 to 12, **characterised in that** the deletion of the L1 protein comprises the nuclear localisation signal (NLS).

14. Virus-like particles according to one of claims 4 to 13, **characterised in that** the particle production from the L1 proteins or L1 and L2 proteins takes place in the cytoplasm.

15. Virus-like particles according to one of claims 4 to 14, **characterised in that** particles are secreted in the supernatant.

16. Virus-like particles according to claim 15, **characterised in that** ca. 5 to 10% of the particles are secreted in the supernatant.

17. Virus-like particles according to one of claims 4 to 16, **characterised in that** the expression of L1 proteins or L1 and L2 proteins takes place in E. coli.

18. Virus-like particles according to claim 17, **characterised in that** 6 histidines have been inserted at the C-terminal deletion in the L1 protein.

19. Virus-like particles according to one of claims 17 and 18, **characterised in that** the production of virus-like particles after expression of L1 proteins or L1 and L2 proteins takes place in E. coli.

20. Virus-like particles according to one of claims 4 to 19, **characterised in that** the further deleted regions in the L1 protein concern the amino acid sequences 311-351, 331-371,391-431 of bovine papilloma virus type 1.

21. Virus-like particles according to claim 20, **characterised in that** the further deleted regions in the L1 protein concern the amino acid sequence 306-315 of human papilloma virus 16.

22. Virus-like particles according to one of claims 1 to 21, **characterised in that** the deleted region of L1 proteins is replaced by other proteins or protein fragments in order to obtain a fusion protein.

23. Virus-like particles according to claim 22, **characterised in that** the proportion of L1 protein is ca. 50 - 99%, preferably ca. 60 - 90%, particularly preferably ca. 80% of the fusion protein.

24. Virus-like particles according to claims 22 and 23, **characterised in that** the deleted region in the L1 protein is replaced by other proteins of papilloma viruses and/or proteins of other origin, whereby virus-like hybrid particles can be produced.

25. Virus-like particles according to one of claims 1 to 24, **characterised in that** the formation of the virus-like particles takes place from an L1 protein, an L1 fusion protein, an L1 protein and L2 protein, an L1 fusion protein and L2 protein, an L1 protein and an L2 fusion protein, or an L1 fusion protein and an L2 fusion protein.

26. Virus-like particles according to one of claims 4 to 25, **characterised in that** at least one of the deleted regions in the L1 protein of a papilloma virus concerns N-terminal amino acid sequences.

27. Virus-like particles according to one of claims 4 to 26, **characterised in that** at least one of the deleted regions in the L1 protein of a papilloma virus concerns amino acid sequences in the middle region of the protein.

28. Fusion proteins for the formation of papilloma virus-like hybrid particles according to one of claims 22 to 25, **characterised in that** the fusion proteins contain deleted L1 proteins of various papilloma viruses and other proteins or fragments of papilloma viruses or of another origin.

29. Fusion proteins for the formation of papilloma virus-like hybrid particles according to one of claims 22 to 25, wherein the fusion proteins contain deleted L1 proteins of various papilloma viruses and other proteins or fragments of papilloma viruses or of another origin, **characterised in that** the other proteins or fragments are early papilloma virus proteins or fragments thereof.

30. Fusion protein according to claim 28 or 29, **characterised in that** the early proteins E1, E2, E4, E5, E6, E7 or fragments thereof are involved.

31. Fusion proteins according to one of claims 28 to 30, **characterised in that** deleted L1 proteins of various human papilloma virus types such as HPV 6, 11, 16, 18, 31, 33, 35, 45 are involved.

32. Process for the expression of the fusion proteins according to one of claims 28 to 31, **characterised in that** the expression of the proteins and fusion proteins is carried out in viral or in eukaryotic vectors.

33. Process for the expression of the fusion proteins and production of papilloma virus-like particles according to one of claims 4 to 31, **characterised in that** the expression of the L1 and/or L2 proteins and fusion proteins is carried out in viral, eukaryotic or prokaryotic vectors.

34. Process according to one of claims 32 and 33, **characterised in that** the L1 and/or L2 proteins or fusion proteins are produced by expression in vaccinia recombinants.

35. Process according to one of claims 32 and 33, **characterised in that** the expression of the L1 and/or L2 proteins and fusion proteins is carried out in baculo viruses or in yeasts.

36. Process according to claim 33, **characterised in that** the expression of the L1 and/or L2 proteins and fusion proteins is carried out in E. coli.

37. Process according to claim 36, **characterised in that** the purification, in particular of the L1 proteins, is carried out by Ni affinity chromatography and renaturation, preferably in 150 mM NaCl, 1 mM CaCl₂, 0.01% Triton-X 100, 10 mM Hepes pH 7.4.

38. Process according to claim 37, **characterised in that** the proteins are present at the time of the purification in denaturation buffer.

39. Process according to claim 38, **characterised in that** the denaturation buffer is 6 M guanidine hydrochloride.

40. Process according to one of claims 33 to 39, **characterised in that** the assembly of the virus-like particles takes place after dialysis.

41. Process according to claim 40, **characterised in that** the dialysis is carried out against 150 mM NaCl, 25mM Ca²⁺, 10 DMSO, 0.1% Triton X 100 and 10 mM tris acetic acid at pH = 5.0.

42. Process according to one of claims 33 to 41, **characterised in that** the particle production takes place in the cytoplasm.

43. Process according to one of claims 33 to 42, **characterised in that** particles are secreted in the supernatant.

44. Process according to claim 43, **characterised in that** ca. 5 to 10% of the particles are secreted in the supernatant.

45. Use of the fusion proteins according to one of claims 28 to 31 for the production of a vaccine for prophylactic and/or therapeutic vaccination, preferably after addition of further components.

46. Use of the virus-like hybrid particles according to one of claims 1 to 27 for the production of a vaccine for prophylactic and/or therapeutic vaccination, preferably after addition of further components.

## Revendications

1. Particules de type virus du papillome, préparées par voie recombinante, qui sont produites après l'expression des protéines de structure virales L1 ou L1 et L2, **caractérisées en ce qu'**une ou plusieurs sections de la protéine L1 sont supprimées, la capacité de formation de particules de type viral étant conservée.

2. Particules de type viral selon la revendication 1, **caractérisées en ce que** les zones supprimées dans la protéine L1 sont les séquences d'acides aminés 311-351, 331-371, 391-431 du virus du papillome bovin de type 1.

3. Particules de type viral selon la revendication 1, **caractérisées en ce que** les zones supprimées dans la protéine L1 représentent la séquence d'acides aminés 306-315 du virus du papillome humain 16.

4. Particules de type virus du papillome, préparées par voie recombinante, qui sont produites après l'expression des protéines de structure virales L1 ou L1 et L2, **caractérisées en ce qu'**une ou plusieurs sections de la protéine L1 sont supprimées, la capacité de formation de particules de type viral étant augmentée par rapport à la formation native et/ou à la production *in vitro.*

5. Particules de type viral selon la revendication 4, **caractérisées en ce qu'**au moins une des zones supprimées dans la protéine L1 d'un virus de papillome représente des séquences d'acides aminés C-terminales.

6. Particules de type viral selon la revendication 5, **caractérisées en ce qu'**au moins une section supprimées dans la zone C-terminale dans la protéine L1 représente une séquence d'acides aminés ayant une longueur d'environ 1 à 34 acides aminés, de préférence d'environ 1 à 26 acides aminés, d'un virus du papillome.

7. Particules de type viral selon l'une des revendications 4 à 6, **caractérisées en ce que**, après insertion en particulier de la suppression C-terminale dans la protéine L1, la production de VLP est augmentée plusieurs fois, de préférence d'au moins 10 fois, particulièrement d'environ 10 à 100 fois.

8. Particules de type viral selon l'une des revendications 4 à 7, **caractérisées en ce que** les zones supprimées dans la protéine L1 représentent 26 acides aminés C-terminaux du virus du papillome bovin.

9. Particules de type viral selon la revendication 8, **caractérisées en ce que** la suppression C-terminale longue de 26 acides aminés (Gly-Ala-Gly-Cys-Ser-Thr-Val-Arg-Lys-Arg-Arg-Ile-Ser-Gln-Lys-Thr-Ser-Ser-Lys-Pro-Ala-Lys-Lys-Lys-Lys-Lys) correspondant à la position des nucléotides 7016 à 7093 GGGGCAGGAT GTTCAACTGT GAGAAAACGA AGAATTAGCC AAAAAACTTC CAGTAAGCCT GCAAAAAAAA AAAAAAAA est insérée dans le CLO L1 du virus du papillome bovin de type 1.

10. Particules de type viral selon l'une des revendications 4 à 7, **caractérisées en ce que** les zones supprimées dans la protéine L1 représentent 34 acides aminés C-terminaux du virus du papillome humain de type 16 (HPV 16).

11. Particules de type viral selon la revendication 10, **caractérisées en ce que** la suppression C-terminale longue de 34 acides aminés (Ala-Gly-Leu-Lys-Ala-Lys-Pro-Lys-Phe-Thr-Leu-Gly-Lys-Arg-Lys-Ala-Thr-Pro-Thr-Thr-Ser-Ser-Thr-Ser-Thr-Thr-Ala-Lys-Arg-Lys-Lys-Arg-Lys-Leu) correspondant à la position des nucléotides 7052 à 7153 GCAGGATTGA AGGCCAAACC AAAATTTACA TTAGGAAAAC GAAAAGCTAC ACCCACCACC TCATCTACCT CTACAACTGC TAAACGCAAA AAACGTAAGC TG est insérée dans le CLO L1 du virus du papillome humain de type 16 (HPV 16).

12. Particules de type viral selon l'une des revendications 5 à 7, **caractérisées en ce qu'**au moins une suppression dans la zone C-terminale dans la protéine L1 représente une séquence d'acides aminés homologue du virus du papillome humain 16 ou d'autres virus du papillome.

13. Particules de type viral selon l'une des revendications 4 à 12, **caractérisées en ce que** la suppression de la protéine L1 comprend le signal de localisation nucléaire (SLN).

14. Particules de type viral selon l'une des revendications 4 à 13, **caractérisées en ce que** la production de particules s'effectue dans le cytoplasme à partir des protéines L1 ou des protéines L1 et L2.

15. Particules de type viral selon l'une des revendications 4 à 14, **caractérisées en ce que** des particules sont sécrétées dans le surnageant.

16. Particules de type viral selon la revendication 15, **caractérisées en ce qu'**environ 5 à 10 % des particules sont sécrétées dans le surnageant.

17. Particules de type viral selon l'une des revendications 4 à 16, **caractérisées en ce que** l'expression des protéines L1 ou des protéines L1 et L2 s'effectue dans *E. coli.*

18. Particules de type viral selon la revendication 17, **caractérisées en ce que** 6 histidines ont été insérées sur la suppression C-terminale dans la protéine L1.

19. Particules de type viral selon l'une des revendications 17 à 18, **caractérisées en ce que** la préparation des VLP s'effectue après l'expression des protéines L1 ou des protéines L1 et L2 dans *E*. *coli.*

20. Particules de type viral selon l'une des revendications 4 à 19, **caractérisées en ce que** les autres zones supprimées dans la protéine L1 représentent les séquences d'acides aminés 311-351, 331-371, 391-431 du virus du papillome bovin de type 1.

21. Particules de type viral selon la revendication 20, **caractérisées en ce que** les autres zones supprimées dans la protéine L1 représentent la séquence d'acides aminés 306-315 du virus du papillome humain 16.

22. Particules de type viral selon l'une des revendications 1 à 21, **caractérisées en ce que** la zone supprimée des protéines L1 est remplacée par d'autres protéines ou fragments de protéines pour obtenir une protéine de fusion.

23. Particules de type viral selon la revendication 22, **caractérisées en ce que** la proportion de protéine L1 atteint environ 50 à 99 %, de préférence environ 60 à 90 %, en particulier environ 80 % de la protéine de fusion.

24. Particules de type viral selon la revendication 22 et 23, **caractérisées en ce que** la zone supprimée dans la protéine L1 est remplacée par d'autres protéines de virus de papillome et/ou des protéines d'une autre origine, moyennant quoi des particules hybrides de type viral peuvent être produites.

25. Particules de type viral selon l'une des revendications 1 à 24, **caractérisées en ce que** la formation des particules de type virales s'effectue à partir d'une protéine L1, d'une protéine de fusion L1, d'une protéine L1 et d'une protéine L2, d'une protéine de fusion L1 et d'une protéine L2, d'une protéine L1 et d'une protéine de fusion L2 ou d'une protéine de fusion L1 et d'une protéine de fusion L2.

26. Particules de type viral selon l'une des revendications 4 à 25, **caractérisées en qu'**au moins une des zones supprimées dans la protéine L1 d'un virus de papillome représente des séquences d'acides aminés N-terminales.

27. Particules de type viral selon l'une des revendications 4 à 26, **caractérisées en qu'**au moins une des zones supprimées dans la protéine L1 d'un virus de papillome représente des séquences d'acides aminés dans la zone médiane de la protéine.

28. Protéines de fusion pour la formation de particules hybrides de type virus du papillome selon l'une des revendications 22 à 25, **caractérisées en ce que** les protéines de fusion contiennent des protéines L1 supprimées de divers virus de papillome et d'autres protéines ou fragments de virus de papillome ou d'autre origine.

29. Protéines de fusion pour la formation de particules hybrides de type virus du papillome selon l'une des revendications 22 à 25, les protéines de fusion contenant des protéines L1 supprimées de divers virus de papillome et d'autres protéines ou fragments de virus de papillome ou d'autre origine, **caractérisées en ce que** les autres protéines ou fragments sont des protéines de virus de papillome précoces ou des fragments de celles-ci.

30. Protéine de fusion selon la revendication 28 ou 29, **caractérisée en ce qu'**il s'agit de protéines précoces E1, E2, E4, E5, E6, E7 ou de fragments de celles-ci.

31. Protéines de fusion selon l'une des revendications 28 à 30, **caractérisées en ce qu'**il s'agit des protéines L1 supprimées de divers types de HPV comme HPV 6, 11, 16, 18, 31, 33, 35, 45.

32. Procédé d'expression de protéines de fusion selon l'une des revendications 28 à 31, **caractérisé en ce que** l'expression des protéines et protéines de fusion est réalisée dans des vecteurs viraux ou eucaryotes.

33. Procédé d'expression des protéines de fusion et de production de particules de type virus de papillome selon l'une des revendications 4 à 31, **caractérisé en ce que** l'expression des protéines L1 et/ou L2 et des protéines de fusion s'effectue dans des vecteurs viraux, eucaryotes ou procaryotes.

34. Procédé selon l'une des revendications 32 ou 33, **caractérisé en ce que** les protéines L1 et/ou L2 ou les protéines de fusion sont produites par expression dans des recombinants de la vaccine.

35. Procédé selon l'une des revendications 32 ou 33, **caractérisé en ce que** l'expression des protéines L1 et/ou L2 et des protéines de fusion se fait dans des baculovirus ou dans des levures.

36. Procédé selon la revendication 33, **caractérisé en ce que** l'expression des protéines L1 et/ou L2 et des protéines de fusion est réalisée dans *E*. *coli.*

37. Procédé selon la revendication 36, **caractérisé en ce que** la purification, en particulier des protéines L1, s'effectue par chromatographie d'affinité Ni et renaturation, de préférence dans 150 mM de NaCl, 1 mM de CaCl₂, 0,01 % de Triton-X 100, 10 mM de Hepes pH 7,4.

38. Procédé selon la revendication 37, **caractérisé en ce que** les protéines se présentent dans un tampon de dénaturation au moment de la purification.

39. Procédé selon la revendication 38, **caractérisé en ce que** le tampon de dénaturation est le chlorhydrate de guanidine à 6 M.

40. Procédé selon l'une des revendications 33 à 39, **caractérisé en ce que** l'assemblage des VLP s'effectue après dialyse.

41. Procédé selon la revendication 40, **caractérisé en ce que** la dialyse s'effectue contre 150 mM de NaCl, 25 mM de Ca²⁺, 10 mM de DMSO, 0,1 % de Triton-X 100 et 10 mM d'acide acétique Tris à pH = 5,0.

42. Procédé selon l'une des revendications 33 à 41, **caractérisé en ce que** la production de particules s'effectue dans le cytoplasme.

43. Procédé selon l'une des revendications 33 à 42, **caractérisé en ce que** des particules sont sécrétées dans le surnageant.

44. Procédé selon la revendication 43, **caractérisé en ce qu'**environ 5 à 10 % des particules sont sécrétées dans le surnageant.

45. Utilisation des protéines de fusion selon l'une des revendications 28 à 31 pour la préparation d'un vaccin destiné à la vaccination prophylactique et/ou thérapeutique, de préférence après addition d'autres composants.

46. Utilisation des particules hybrides de type virus selon l'une des revendications 1 à 27 pour la préparation d'un vaccin destiné à la vaccination prophylactique et/ou thérapeutique, de préférence après addition d'autres composants.
